# EUROPEAN PATENT APPLICATION

(11) **EP 1 898 335 A1**
(43) Date of publication of application: **12.03.2008**
(21) Application number: 06076689.6
(22) Date of filing: 07.09.2006
(51) Int. Cl.: G06F 21/24

(54) **Method and system for verifying the equality of a first secret code and a second secret code without revealing the first secret code and the second secret code**

(71) Applicant: Emotional Brain B.V., 1311 RL Almere (NL)
(72) Inventor: Lebbink, Henk-Jan, 1053 JL Amsterdam (NL)
(74) Representative: van Loon, C.J.J.

(57) **Abstract**

System and method for verifying the equality or inequality of a first and second secret code held by a first and second storage unit, respectively, without revealing the first and second secret code. The method comprises generating by the first and second storage unit, respectively, a first and second key from which the first and second secret code cannot be deduced; transmitting the first and second key to the second and first storage unit, respectively; generating on the basis of the first secret code and the second key a first combination key; generating on the basis of the second secret code and the first key a second combination key; comparing the first combination key with the second combination key; and concluding the equality or inequality of the first secret code and the second secret code when the first combination key and the second combination key are equal or non-equal, respectively.

## Description

The invention relates to a method for verifying by a verification unit the equality or inequality of a first secret code held by a first storage unit and a second secret code held by a second storage unit without revealing the first secret code to the verification unit and/or the second storage unit and without revealing the second secret code to the verification unit and/or the first storage unit.

The invention further relates to a system for verifying the equality of a first secret code and a second secret code without revealing the first secret code and the second secret code, a verification unit of the system, a storage unit of the system and a computer program product including program code portions for performing, when run on a programmable apparatus, a method according to the invention.

Comparing secret codes by a verification unit is known per se. Known methods for verifying by a verification unit the equality or inequality of secret codes are e.g. based on encrypting the secret codes before being compared. These methods have the disadvantage that the encrypted secret codes may be decrypted by unauthorised persons or devices, which may lead to breach of the secrecy of the secret codes. Hence, secrecy of the secret codes cannot be fully guaranteed. Further, said encrypting may require great computing time and/or power in order to at least provide reasonable protection of secrecy. Some known methods rely on the first storage unit and the second storage unit to mutually agree upon a key to be used encrypting the secret codes and/or in transferring data to the verification unit. Such methods may suffer the disadvantage that the first and/or second storage unit may cheat, i.e. on purpose use a different key than agreed upon in the process, to gain information on the secret code of the other, without revealing its own secret code.

It is an object of the invention to at least reduce one of the above disadvantages.

Thereto, according to the invention, a method according to claim 1 is provided. This provides the advantage that the first storage unit only has to transmit the first key from which the first secret code cannot be deduced to the second storage unit, and the second storage unit only has to transmit the second key from which the first secret code cannot be deduced to the second storage unit. The first and second key may be chosen, e.g. randomly, by the first and second storage unit respectively. Hence, the first and second storage unit do not reveal their respective secret codes to each other. Further, the first and second storage unit only need to transmit the first and the second combination key, respectively, to the verification unit. Hence a very simple method is obtained which requires limited communication between the first storage unit, second storage unit and verification unit.

Here the terms "code" and "key" encompass any symbol or combination of symbols comprising information such as binary, numerical, alphanumerical or special characters.

In an embodiment the method comprises generating by the first storage unit a third key on the basis of the first secret code and the first key, such that the first key and the third key in combination uniquely represent the first secret code and/or generating by the second storage unit a fourth key on the basis of the second secret code and the second key, such that the second key and the fourth key in combination uniquely represent the second secret code, and generating the first combination key on the basis of the third key and the second key and/or generating the second combination key on the basis of the fourth key and the first key. Thus, it can easily be made sure that the equality of the first and second combination key uniquely determines the equality of the first and second secret codes.

Preferably third key and the first key are generated such that the first secret code cannot be deduced from the third key alone or from the first key alone, and/or the fourth key and the second key are generated such that the second secret code cannot be deduced from the fourth key alone or from the second key alone. Hence, neither the verification unit, nor the second storage unit gains information from the first storage unit from which the first secret code can be deduced. Also, neither the verification unit, nor the first storage unit gains information from the second storage unit from which the second secret code can be deduced.

Preferably the third key is generated by performing an exclusive or operation with the first secret code and the first key and/or the fourth key is generated by performing an exclusive or operation with the second secret code and the second key. Hence, it is easily achieved that the first secret code cannot be deduced from the third key alone or from the first key alone, and/or the second secret code cannot be deduced from the fourth key alone or from the second key alone. Further, it is easily achieved that the first and third key in combination uniquely represent the first secret code and/or the second key and the fourth key in combination uniquely represent the second secret code.

Preferably the first combination key is generated by performing an exclusive or operation with the third key and the second key and/or the second combination key is generated by performing an exclusive or operation with the fourth key and the first key. Hence, it is achieved that the first secret code and the second secret code cannot be deduced from the first combination key and/or from the second combination key.

Preferably the third key is kept secret for the second storage unit and/or the verification unit and/or the fourth key is kept secret for the first storage unit and/or the verification unit and/or the first key is kept secret for the verification unit and/or the second key is kept secret for the verification unit. Hence, neither the verification unit, nor the second storage unit gains information from the first storage unit from which the first secret code can be deduced. Also, neither the verification unit, nor the first storage unit gains information from the second storage unit from which the second secret code can be deduced.

In an embodiment the method further comprises keeping the equality or inequality of the first and second secret code secret for the first storage unit and the second storage unit. Hence, the first storage unit and second storage unit cannot deduce any information regarding the secret code of the other.

It is possible that the first storage unit comprises a first database holding a plurality of first secret codes and the second storage unit comprises a second database holding a plurality of second secret codes. Hence it is possible to verify the equality or inequality of a plurality of secret codes.

In an embodiment the method comprises generating by the first storage unit a plurality of first combination keys, each corresponding to a respective first secret code, and generating by the second storage unit a plurality of second combination keys, each corresponding to a respective second secret code; transmitting at least one first combination key of the plurality of first combination keys and at least one second combination key of the plurality of second combination keys to the verification unit; comparing, by the verification unit, the at least one first combination key with the at least one second combination key; and concluding the equality of at least one first secret code held by the first storage unit and at least one second secret code held by the second storage unit when the at least one first combination key equals the at least one second combination key, and concluding the inequality of at least one first secret code held by the first storage unit and at least one second secret code held by the second storage unit when the at least one first combination key does not equal the at least one second combination key. Hence, a simple method is provided for verifying the equality or inequality of at least one first secret code of a plurality of secret codes to at least one second secret code of a plurality of second secret codes.

In an embodiment, the method comprises concluding the inequality of at least one first secret code held by the first storage unit and the plurality of second secret codes held by the second storage unit when the at least one first combination key does not equal any second combination key of the plurality of second combination keys. Hence, a simple method is provided for verifying the inequality of at least one first secret code to any second secret code of a plurality of second secret codes.

In an embodiment, the method comprises comparing, by the verification unit, each first combination key of the plurality of first combination keys with each second combination key of the plurality of combination keys; and concluding the equality of at least one first secret code held by the first storage unit and at least one second secret code held by the second storage unit when at least one first combination key equals at least one second combination key, and concluding the inequality of at least one first secret code held by the first storage unit and the plurality of second secret codes held by the second storage unit when at least one first combination key does not equal any second combination key of the plurality of second combination keys. Hence, a simple method is provided for comparing any first secret code of a plurality of first secret code with any second secret code of a plurality of second secret codes.

It is possible, that the method comprises dividing the first secret code into a plurality of first secret subcodes and dividing the second secret code into a plurality of second secret subcodes, generating by the first storage unit a plurality of first subkeys, each corresponding to a respective first secret subcode, and generating by the second storage unit a plurality of second subkeys, each corresponding to a respective second secret subcode; transmitting the at least one first subkey from the first storage unit to the second storage unit; transmitting the at least one second subkey from the second storage unit to the first storage unit; generating by the first storage unit a plurality of first combination subkeys, each corresponding to a respective first secret subcode and second subkey, and generating by the second storage unit a plurality of second combination subkeys, each corresponding to a respective second secret subcode and first subkey; transmitting at least one first combination subkey and at least one second combination subkey to the verification unit; comparing, by the verification unit, the at least one first combination subkey with the respective at least one second combination subkey; and concluding the equality of the first secret code and the second secret code unit when all first combination subkeys equal the respective second combination subkeys, and concluding the inequality of the first secret code and the second secret code when at least one first combination subkey does not equal the respective second combination subkey. This may provide the advantage that large secret codes may be divided into smaller secret subcodes, which may be compared separately, e.g. sequentially and/or bit-wise. Hence, it is also possible to conclude the inequality of a first secret code and a second secret code on the basis of the inequality of a first secret subcode and a respective second secret subcode. Thus, it is possible to conclude the inequality when not the entire first secret code has been compared to the entire second secret code. Hence, transmission of data may be limited. Thus the concluding of the inequality may be performed faster than when the entire first and second secret code are compared.

It is possible, that a plurality of first secret codes forms a first secret supercode and a plurality of second secret codes forms a second secret supercode, and the method comprises verifying the equality or inequality of at least one first secret code of the plurality of first secret codes to at least one respective second secret code of the plurality of second secret codes, until the equality of all first secret codes with all respective second secret codes has been concluded, in which case the equality of the first secret supercode to the second secret supercode is concluded, or until the inequality of at least one first secret code with the respective second secret code has been concluded, in which case the inequality of the first secret supercode to the second secret supercode is concluded. Hence it is possible to simply and efficiently verify the equality or inequality of records which contain a plurality of secret codes. An example of such record may for instance be a medical record or criminal record.

In an embodiment the verification unit, the first storage unit and the second storage unit are connectable via a network, such as the internet. Hence, the verifying of the equality or inequality may be performed by a verification unit which is remote from the first and/or second storage unit. Further, the first and second storage unit may be remote from each other.

Further details, aspects and embodiments of the invention will be described, by way of example only, with reference to the drawings, wherein
Fig. 1 illustrates a first embodiment of a system for verifying the equality or inequality of a first and second secret code according to the invention;
Fig. 2 illustrates a second embodiment of a system for verifying the equality or inequality of a first and second secret code according to the invention;
Fig. 3 illustrates a third embodiment of a system for verifying the equality or inequality of a first and second secret code according to the invention; and
Fig. 4 illustrates an embodiment of a system for verifying the equality or inequality of a first and second secret supercode.

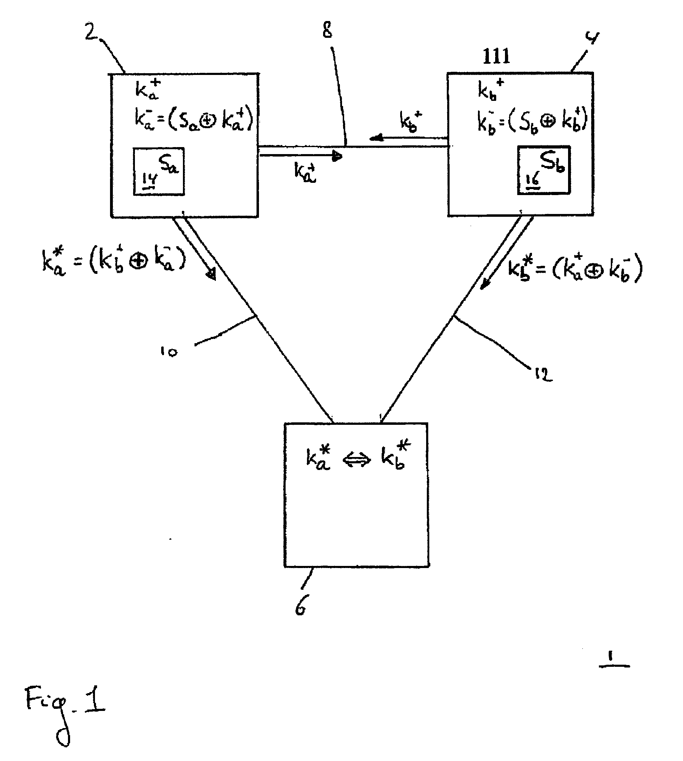

Fig. 1 illustrates a first embodiment of a system 1 for verifying the equality or inequality of a first and second secret code, Sₐ,S_{b}, without revealing the first secret code Sₐ and the second secret code S_{b}, according to the invention. The system 1 comprises a first storage unit 2, a second storage unit 4 and a verification unit 6. The first storage unit 2 is communicatively connected to the second storage unit 4 via a first communications link 8, e.g. via the internet. The first storage unit 2 is communicatively connected to the verification unit 6 via a second communications link 10, e.g. via the internet. The second storage unit 4 is communicatively connected to the verification unit 6 via a third communications link 12, e.g. via the internet.

The first storage unit 2 holds the first secret code Sₐ, e.g. in a first computer memory 14, such as a database, of the first storage unit 2. The second storage unit 4 holds the second secret code S_{b}, e.g. in a second computer memory 16, such as a database, of the second storage unit 4.

The system 1 is arranged for performing a method for verifying by the verification unit 6 the equality or inequality of a first secret code Sₐ held by the first storage unit 2 and the second secret code S_{b} held by the second storage unit 4 without revealing the first secret code Sₐ to the verification unit 6 and/or the second storage unit 4 and without revealing the second secret code S_{b} to the verification unit 6 and/or the first storage unit 2.

The system 1 described thus far, can be operated according to the following method.

The first storage unit 2 generates a first key kₐ⁺ from which the first secret code Sₐ cannot be deduced. The first key kₐ⁺ may for instance be a key randomly chosen by the first storage unit 2. The second storage unit 4 generates a second key k_{b}⁺ from which the second secret code S_{b} cannot be deduced. The second key k_{b}⁺ may for instance be a key randomly chosen by the second storage unit 4. Next, the first storage unit 2 transmits the first key kₐ⁺ to the second storage unit 4, and the second storage unit 4 transmits the second key k_{b}⁺ to the first storage unit 2.

In this example, the first storage unit 2 generates a third key kₐ⁻ on the basis of the first secret code Sₐ and the first key kₐ⁺. In this example the third key kₐ⁻ is generated by performing an exclusive or operation with the first secret code Sₐ and the first key kₐ⁺ (denoted as kₐ⁻=kₐ⁺⊕Sₐ in Fig. 1). Hence, in this example, the first key kₐ⁺ and the third key kₐ⁻ in combination uniquely represent the first secret code Sₐ, and the first secret code Sₐ cannot be deduced from the third key ka⁻ alone or from the first key kₐ⁺ alone. Using the exclusive or operation has the additional advantage that the first key and the third key may have the same size (e.g. counted in bits) as the first secret code, while still the first secret code cannot be deduced from the third key alone or from the first key alone. In prior art encryption schemes often long keys are used (longer than the secret code to be represented), while still it is possible, albeit with much effort, to deduce the secret code from said key.

Further, in this example, the first storage unit 2 generates a first combination key kₐ* by performing an exclusive or operation with the third key kₐ⁻ and the second key k_{b}⁺, such that kₐ*=k_{b}⁺⊕kₐ⁻. Hence the first combination key kₐ* is generated on the basis of the third key kₐ⁻ and the second key k_{b}⁺. More in general, in holds that the first storage unit 2 generates a first combination key kₐ* on the basis of the first secret code Sₐ and the second key k_{b}⁺.

In this example, the second storage unit 4 generates a fourth key k_{b}on the basis of the second secret code S_{b} and the second key k_{b}⁺. In this example the third key k_{b}⁻ is generated by performing an exclusive or operation with the second secret code S_{b} and the second key k_{b}⁺ (denoted as k_{b}⁻=k_{b} ⁺⊕S_{b} in Fig. 1). Hence, in this example, the second key k_{b}⁺ and the fourth key k_{b}⁻ in combination uniquely represent the second secret code S_{b}, and the second secret code S_{b} cannot be deduced from the fourth key k_{b}⁻ alone or from the second key k_{b}⁺ alone.

Further, in this example, the second storage unit 4 generates a second combination key k_{b}* by performing an exclusive or operation with the fourth key k_{b}⁻ and the first key kₐ⁺, such that k_{b}*=kₐ⁺⊕k_{b}⁻. Hence the second combination key k_{b}* is generated on the basis of the fourth key k_{b}⁻ and the first key kₐ⁺. More in general, it holds that the second storage unit 4 generates the second combination key k_{b}* on the basis of the second secret code S_{b} and the first key ka⁺.

Next, the first storage unit 2 transmits the first combination key kₐ* to the verification unit 6, and the second storage unit 4 transmits the second combination key k_{b}* to the verification unit 6. The verification unit 6 compares the first combination key kₐ* with the second combination key k_{b}*. If the verification unit 6 determines that the first combination key kₐ* equals the second combination key k_{b}*, the verification unit 6 concludes the equality of the first secret code Sₐ held by the first storage unit 2 and the second secret code S_{b} held by the second storage unit 4. If the verification unit 6 determines that the first combination key kₐ* does not equal the second combination key k_{b}*, the verification unit 6 concludes the inequality of the first secret code Sₐ held by the first storage unit 2 and the second secret code S_{b} held by the second storage unit 4.

As, in this example the first secret code Sₐ cannot be deduced from the third key kₐ⁻ alone or from the first key kₐ⁺ alone, neither the second storage unit 4, nor the verification unit 6 can deduce the first secret code Sₐ from the information transmitted by the first storage unit 2 to the second storage unit 4 or the verification unit 6, respectively. Preferably, the third key kₐ⁻ is kept secret for the verification unit 6 and the first combination key kₐ* is kept secret for the second storage unit 4. In that case neither the second storage unit 4, nor the verification unit 6 can deduce the first secret code Sₐ.

As, in this example the second secret code S_{b} cannot be deduced from the fourth key k_{b} alone or from the second key k_{b}⁺ alone, neither the first storage unit 2, nor the verification unit 6 can deduce the second secret code S_{b} from the information transmitted by the second storage unit 4 to the first storage unit 2 or the verification unit 6, respectively. Preferably, the fourth key k_{b}⁻ is kept secret for the verification unit 6 and the second combination key k_{b}* is kept secret for the first storage unit 2. In that case neither the first storage unit 2, nor the verification unit 6 can deduce the second secret code S_{b}.

In this example, the verification unit 6, keeps the equality or inequality of the first and second secret code Sₐ,S_{b} secret for the first storage unit 2 and the second storage unit 4. In that case no information about the first and second secret code Sₐ,S_{b} is learned by the first storage unit and the second storage unit.

The method described above may be elaborated as follows. The verification unit 6 requests the first combination key kₐ* from the first storage unit 2, and the second combination key k_{b}* from the second storage unit. The first storage unit 2 requests the second key k_{b}⁺ from the second storage unit 4 and the second storage unit 4 requests the first key kₐ⁺ from the first storage unit 2. The first storage unit 2 generates and transmits the first key kₐ⁺ to the second storage unit 4 after receiving the request for the first key kₐ⁺. The second storage unit 4 generates and transmits the second key k_{b}⁺ to the first storage unit 2 after receiving the request for the second key k_{b}⁺. After receiving the second key k_{b}⁺, the first storage unit 2 determines the first combination key kₐ* on the basis of the first secret code Sₐ and the second key k_{b}⁺. After receiving the first key kₐ⁺, the second storage unit 4 determines the second combination key k_{b}* on the basis of the second secret code S_{b} and the first key kₐ⁺. The first storage unit 2 transmits the first combination key kₐ* to the verification unit 6. The second storage unit 4 transmits the second combination key k_{b}* to the verification unit 6. The verification unit compares the first combination key kₐ* and the second combination key k_{b}*.

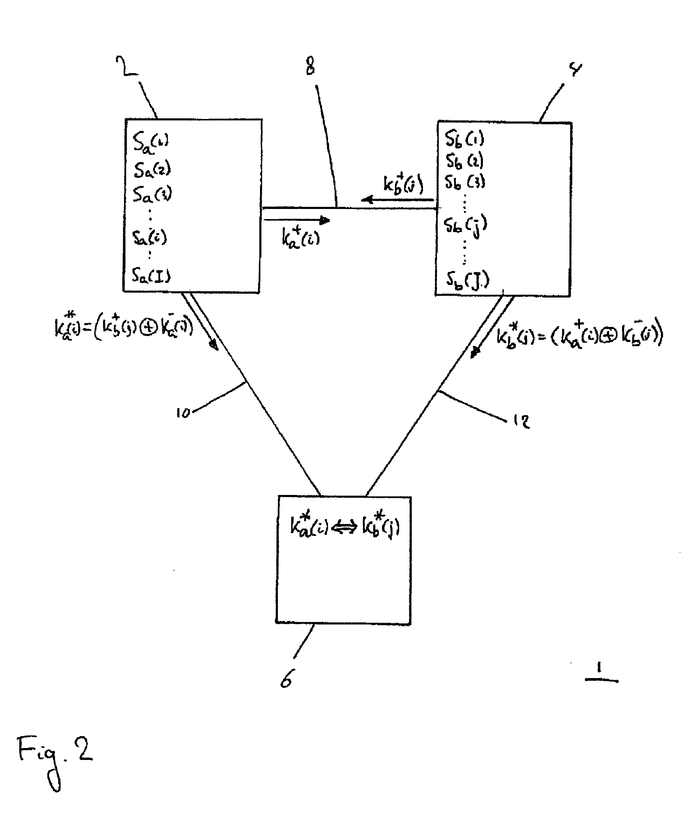

Fig. 2 illustrates a second embodiment of the system 1 for verifying the equality or inequality of the first and second secret code according to the invention. In the example of Fig. 2, the first storage unit 2 comprises a first database holding a plurality of first secret codes Sₐ(i) (i=1,2,...I) and a second database holding a plurality of second secret codes S_{b}(j) (j=1,2,...J). In this example, the plurality of first secret codes forms a first privacy protected dataset and the plurality of second secret codes forms a second privacy protected dataset. The privacy may e.g. be protected by law or by a code of conduct.

In this example, the plurality of first secret codes is a plurality of personal identification numbers, such as social security numbers, of truants in a high-school database. In this example the plurality of second secret codes is a plurality of personal identification numbers, such as social security numbers, of juvenile delinquents, e.g. in a police or court database. In order to identify high-risk juveniles, without revealing any data from either the high-school database or the police database, first the equality of any first secret code comprised in the first dataset to any second secret code comprised in the second dataset must be verified. As a second step, the content of the secret codes which have been identified as present in both datasets, but of which the content is still secret, may, be revealed e.g. to a social work department. In this second step high levels of security may be used to protect privacy of the secret codes to be revealed.

It will be appreciated that in an advanced embodiment, the system 1 will be arranged to keep the equality or inequality of the secret codes secret to the first storage unit and the second storage unit, to prevent the storage units from gaining any information on the secret code(s) of the other. Thereto, the verification unit may be arranged to keep the results of the verification secret, e.g. in a password protected area of the verification unit. It will be appreciated that this advanced feature can be implemented in the examples of Figs.1 and 2 and Figs. 3 and 4 to follow.

The first storage unit 2 generates a plurality of first keys kₐ⁺(i), each corresponding to one respective first secret code Sₐ(i), such that the first secret code Sₐ(i) cannot be deduced from the respective first key kₐ⁺(i). Thus, a first first key kₐ⁺(1) corresponds to a first first secret code Sₐ(1), a second first key kₐ⁺(2) corresponds to a second first secret code Sₐ(2), a third first key kₐ⁺(3) corresponds to a third first secret code Sₐ(3), etc. In this example the first storage unit 2 also generates a plurality of third keys kₐ⁻(i), each corresponding to a respective first secret code Sₐ(i). The second storage unit 4 generates a plurality of second keys k_{b}⁺(j), each corresponding to one respective second secret code S_{b}(j), such that the second secret code S_{b}(j) cannot be deduced from the respective second key k_{b}⁺(j). In this example the second storage unit 4 also generates a plurality of fourth keys k_{b}⁻(j), each corresponding to a respective second secret code S_{b}(j).

The first storage unit generates a plurality of first combination keys kₐ*(i), each corresponding to a respective first secret code Sₐ(i), in this example by calculating the exclusive or kₐ*(i)= k_{b}⁺(i)⊕kₐ⁻(i). The second storage unit 4 generates a plurality of second combination keys k_{b}*(j), each corresponding to a respective second secret code S_{b}(j), in this example by calculating the exclusive or k_{b}*(j)₌ kₐ⁺(j)⊕k_{b}⁻(j).

The first storage 2 unit transmits at least one first combination key kₐ*(i) of the plurality of first combination keys to the verification unit 6. The second storage unit 4 transmits at least one second combination key k_{b}*(j) of the plurality of second combination keys to the verification unit 6. Then, the verification unit 6 compares the at least one first combination key kₐ*(i) with the at least one second combination key k_{b}*(j). If the at least one first combination key kₐ*(i) equals the at least one second combination key k_{b}*(j) the verification unit 6 concludes the equality of the first secret code Sₐ(i) corresponding the first combination key kₐ*(i) and the second secret code S_{b}(j) corresponding to the second combination key k_{b}*(j). If the at least one first combination key kₐ*(i) does not equal the at least one second combination key k_{b}*(j) the verification unit 6 concludes the inequality of the first secret code Sₐ(i) corresponding the first combination key kₐ*(i) and the second secret code S_{b}(j) corresponding to the second combination key k_{b}*(j). However, in this case the first secret code Sₐ(i) may still be equal to an other second secret code S_{b}(j). Hence, in a special embodiment the second storage unit will keep transmitting second combination keys until a second combination key is transmitted that is equal to the first combination key or until all second combination keys have been transmitted. In the latter case, when the first combination key does not equal any second combination key of the plurality of second combination keys, the verification unit will conclude the inequality of the first secret code held by the first storage unit and the plurality of second secret codes held by the second storage unit.

In an elaborate embodiment, the verification unit compares each first combination key of the plurality of first combination keys with each second combination key of the plurality of combination keys, and concludes the equality of at least one first secret code held by the first storage unit and at least one second secret code held by the second storage unit when at least one first combination key equals at least one second combination key, and concluding the inequality of at least one first secret code held by the first storage unit and the plurality of second secret codes held by the second storage unit when at the least one first combination key does not equal any second combination key of the plurality of second combination keys.

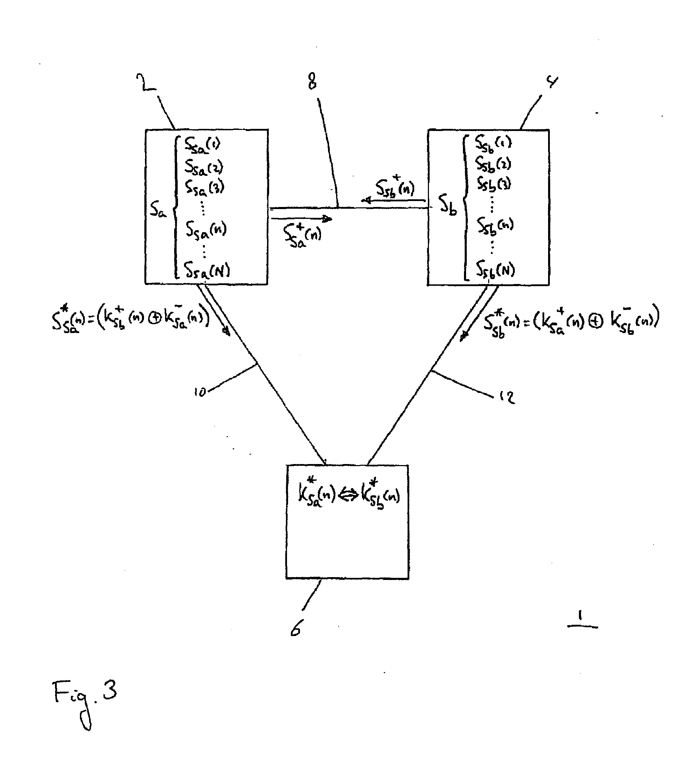

Fig. 3 illustrates a third embodiment of the system 1 for verifying the equality or inequality of the first and second secret code according to the invention. In the example of Fig. 3 the first secret code Sₐ is divided into a plurality of first secret subcodes Ssₐ(n) (n=1,2,3,...,N) and the second secret code S_{b} is divided into a plurality of second secret subcodes Ss_{b}(n). In this example, the first secret code Sₐ and the second secret code S_{b} are divided into separate bits. It will be appreciated that the number of first secret subcodes, here N, must equal the number of second secret subcodes for the first and second secret code to be able to be equal.

The first storage unit 2 generates a plurality of first subkeys k_{Sa}⁺(n), each corresponding to a respective first secret subcode S_{Sa}(n). The second storage unit 4 generates a plurality of second subkeys k_{Sb}⁺(n), each corresponding to a respective second secret subcode S_{Sb}(n). In this example the first storage 2 unit transmit the first first subkey k_{Sa}⁺(1) to the second storage unit and the second storage unit 4 transmits the first second subkey k_{Sb}⁺(1) to the first storage unit 2.

The first storage unit 2 generates a first first combination subkey k_{Sa}*(1), corresponding to the first first secret subcode S_{Sa}(1) and the first second subkey k_{Sb}⁺(1). The second storage unit 4 generates a first second combination subkeys k_{Sb}*(1) corresponding to the first second secret subcode S_{Sb}(1) and the first first subkey k_{Sa}⁺(1). The first storage unit transmits the first first combination subkey k_{Sa}*(1) to the verification unit 6. The second storage unit transmits the first second combination subkey k_{Sb}*(1) to the verification unit 6.

The verification unit 6 compares the first first combination subkey ksₐ*(1) with first second combination subkey k_{Sb}*(1). If the first first combination subkey k_{Sa}*(1) does not equal the first second combination subkey k_{Sb}*(1), the verification unit will conclude the inequality of the first first secret subcode S_{Sa}(1) and the first second secret subcode S_{Sb}(1). Based on this inequality, the verification unit will also conclude the inequality of the first secret code Sₐ and the second secret code S_{b}.

If the first first combination subkey k_{Sa}*(1) equals the first second combination subkey k_{Sb}*(1), the verification unit will conclude the equality of the first first secret subcode S_{Sa}(1) and the first second secret subcode S_{Sb}(1). The verification unit will now request a second first combination subkey k_{Sa}*(2) and a second second combination subkey k_{Sb}*(2) from the first and second storage unit respectively. If the second first combination subkey k_{Sa}*(2) does not equal the second second combination subkey k_{Sb}*(2), the verification unit will conclude the inequality of the first secret code Sₐ and the second secret code S_{b}. If the second first combination subkey k_{Sa}*(2) equals the second second combination subkey k_{Sb}*(2), the verification unit will conclude the equality of the second first secret subcode Ssa(2) and the second second secret subcode S_{Sb}(2). The verification unit will now request a third first combination subkey k_{Sa}*(3) and a third second combination subkey k_{Sb}*(3). This process will be repeated until either the inequality of the first and second secret code Sₐ,S_{b} is concluded, or until all first combination subkeys k_{Sa}*(n) and second combination subkeys k_{sb}*(n) associated with the plurality of first subkeys k_{Sa}⁺(n) and second subkeys k_{Sb}⁺(n) have been compared. The equality of the first secret code Sₐ and the second secret code S_{b} will be concluded when all first combination subkeys k_{Sa}*(n) equal the respective second combination subkeys k_{Sb}*(n). It will be appreciated that when the secret codes are divided into secret subcodes, concluding the inequality of two secret codes may not require comparing the entire secret codes, but only portions of them. This may save processing time and data traffic.

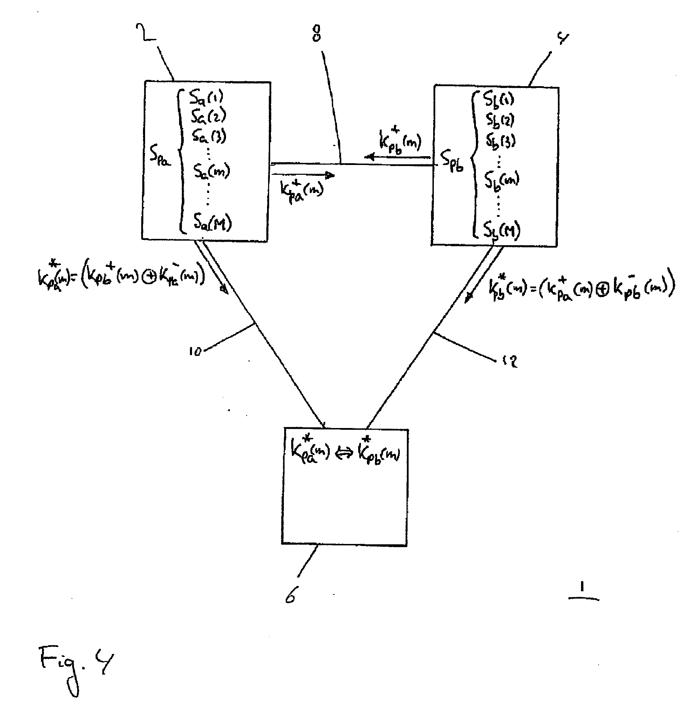

Fig. 4 illustrates an embodiment of the system 1 for verifying the equality or inequality of a first and second secret supercode. In the example of Fig. 4 a plurality of first secret codes Sₐ(m) (m=1,2,3,..., M) forms a first secret supercode S_{Pa} and a plurality of second secret codes S_{b}(m) forms a second secret supercode S_{Pb}. The supercodes may for instance be medical records, comprising secret codes such as name information, an identification number (such as a social security number), blood pressure value, medicine prescriptions, etc.

The first storage unit 2 generates a plurality of first superkeys k_{Pa}⁺(m), each corresponding to a respective first secret code Sₐ(m) of the first secret supercode S_{Pa}. The second storage unit 4 generates a plurality of second superkeys k_{Pb}⁺(m), each corresponding to a respective second secret code S_{b}(m) of the second secret supercode S_{Pb}. In this example the first storage 2 unit transmit the first first superkey k_{Pa}⁺(1) to the second storage unit and the second storage unit 4 transmits the first second superkey k_{Pb}⁺(1) to the first storage unit 2.

The first storage unit 2 generates a first first combination superkey k_{Pa}*(1), corresponding to the first secret code Sₐ(1) and the first second superkey k_{Pb}⁺(1). The second storage unit 4 generates a first second combination superkeys k_{Pb}*(1) corresponding to the first second secret code S_{b}(1) and the first first superkey k_{Pa}⁺(1). The first storage unit transmits first first combination superkey k_{Pa}*(1) to the verification unit 6. The second storage unit transmits the first second combination superkey k_{Pb}*(1) to the verification unit 6.

The verification unit 6 compares the first first combination superkey k_{Pa}*(1) with first second combination superkey k_{Pb}*(1). If the first first combination superkey k_{Pa}*(1) does not equal the first second combination superkey k_{Pb}*(1), the verification unit will conclude the inequality of the first first secret code Sₐ(1) and the first second secret code S_{b}(1). Based on this inequality, the verification unit will also conclude the inequality of the first secret supercode S_{Pa} and the second secret supercode S_{Pb}.

If the first first combination superkey k_{Pa}*(1) equals the first second combination superkey k_{Pb}*(1), the verification unit will conclude the equality of the first first secret code Sₐ(1) and the first second secret code S_{b}(1). The verification unit will now request a second first combination superkey k_{Pa}*(2) and a second second combination superkey k_{Pb}*(2) from the first and second storage unit respectively. If the second first combination superkey k_{Pa}*(2) does not equal the second second combination superkey k_{Pb}*(2), the verification unit will conclude the inequality of the first secret supercode S_{Pa} and the second secret supercode S_{Pb}. If the second first combination superkey k_{Pa}*(2) equals the second second combination superkey k_{Pb}*(2), the verification unit will conclude the equality of the second first secret code Sₐ(2) and the second second secret code S_{b}(2). The verification unit will now request a third first combination superkey k_{Pa}*(3) and a third second combination superkey k_{Pb}*(3). This process will be repeated until either the inequality of the first and second secret supercode S_{Pa},S_{Pb} is concluded, or until all first combination superkeys k_{Pa}*(m) and second combination superkeys k_{Pb}*(m) associated with the plurality of first superkeys k_{Pa}⁺(m) and second superkeys k_{Pb}⁺(m) have been compared. The equality of the first secret supercode S_{Pa} and the second secret supecode S_{Pb} will be concluded when all first combination superkeys k_{Pa}*(m) equal the respective second combination superkeys k_{Pb}*(m).

In the foregoing specification, the invention has been described with reference to specific examples of embodiments of the invention. It will, however, be evident that various modifications and changes may be made therein without departing from the broader spirit and scope of the invention as set forth in the appended claims. For example, in the examples, the first storage unit generates the first combination key on the basis of the second key and the third key. It will be appreciated that it is also possible that the first storage unit generates the first combination key directly from the second key and the first secret code, e.g. as the exclusive or of the first secret code with the result of the exclusive or of the first key and the second key.

In the example of Fig. 3, the first storage unit generates a plurality of first subkeys. It will be appreciated that it is also possible that the first storage unit starts by generating only one first subkey and only generates a next first subkey if the first secret subcode has been found to equal the second secret subcode. The same may apply to the second storage unit. It will be appreciated, that the same may apply, mutatis mutandis, to the examples of Figs. 2 and 4.

In the example of Fig. 3 the first secret code is divided into a plurality of first secret subcodes. For each first secret subcode a first subkey is generated. It will be appreciated that it is possible that more subkeys are generated than there are secret subcodes. In that case the plurality of subkeys may contain dummy subkeys. In an embodiment the plurality of first and second subkeys may contain a predetermined number of first and second subkeys, respectively. Hence, it is possible to compare secret codes of different lengths (different numbers of secret subcodes), e.g. without revealing that the secret codes have different lengths. It will be appreciated, that the same may apply, mutatis mutandis, to the example of Fig. 4.

In the examples the exclusive or operation is used to determine the third key from the first secret code and the first key. It will be appreciated that other operations can be used to determine the third key. It is e.g. possible to determine the third key as the numerical sum of the (chosen) first key and a numerical value of the first secret code and the fourth key as the numerical sum of the (chosen) second key and the inverse of a numerical value of the second secret code. The first combination key can then be defined as the third key minus the second key, and the second combination key can be defined as the first key minus the fourth key.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other items or steps then those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. Method for verifying by a verification unit the equality or inequality of a first secret code held by a first storage unit and a second secret code held by a second storage unit without revealing the first secret code to the verification unit and/or the second storage unit and without revealing the second secret code to the verification unit and/or the first storage unit, comprising
generating by the first storage unit a first key from which the first secret code cannot be deduced;
generating by the second storage unit a second key from which the second secret code cannot be deduced;
transmitting the first key from the first storage unit to the second storage unit;
transmitting the second key from the second storage unit to the first storage unit;
generating by the first storage unit on the basis of the first secret code and the second key a first combination key;
generating by the second storage unit on the basis of the second secret code and the first key a second combination key;
transmitting the first combination key and the second combination key to the verification unit;
comparing, by the verification unit, the first combination key with the second combination key; and
concluding the equality of the first secret code held by the first storage unit and the second secret code held by the second storage unit when the first combination key equals the second combination key, and
concluding the inequality of the first secret code held by the first storage unit and the second secret code held by the second storage unit when the first combination key does not equal the second combination key.

2. Method according to claim 1, comprising
generating by the first storage unit a third key on the basis of the first secret code and the first key, such that the first key and the third key in combination uniquely represent the first secret code and/or
generating by the second storage unit a fourth key on the basis of the second secret code and the second key, such that the second key and the fourth key in combination uniquely represent the second secret code, and
generating the first combination key on the basis of the third key and the second key and/or
generating the second combination key on the basis of the fourth key and the first key.

3. Method according to claim 2, wherein the third key and the first key are generated such that the first secret code cannot be deduced from the third key alone or from the first key alone, and/or wherein the fourth key and the second key are generated such that the second secret code cannot be deduced from the fourth key alone or from the second key alone.

4. Method according to claim 2 or 3, wherein the third key is generated by performing an exclusive or operation with the first secret code and the first key and/or wherein the fourth key is generated by performing an exclusive or operation with the second secret code and the second key.

5. Method according to any one of claims 2-4, wherein the first combination key is generated by performing an exclusive or operation with the third key and the second key and/or wherein the second combination key is generated by performing an exclusive or operation with the fourth key and the first key.

6. Method according to any one of claims 2-5, wherein the third key is kept secret for the second storage unit and/or the verification unit and/or
wherein the fourth key is kept secret for the first storage unit and/or the verification unit and/or wherein the first key is kept secret for the verification unit and/or wherein the second key is kept secret for the verification unit.

7. Method according to any one of the preceding claims, wherein the method further comprises:
keeping the equality or inequality of the first and second secret code secret for the first storage unit and the second storage unit.

8. Method according to any one of the preceding claims, wherein the first key is chosen by the first storage unit and/or the second key is chosen by the second storage unit.

9. Method according to any one of the preceding claims, wherein the first storage unit comprises a first computer memory, such as a database and/or wherein the second storage unit comprises a second computer memory, such as a database.

10. Method according to any one of the preceding claims, wherein the first storage unit comprises a first database holding a plurality of first secret codes and the second storage unit comprises a second database holding a plurality of second secret codes.

11. Method according to claim 10, comprising
generating by the first storage unit a plurality of first combination keys, each corresponding to a respective first secret code, and generating by the second storage unit a plurality of second combination keys, each corresponding to a respective second secret code;
transmitting at least one first combination key of the plurality of first combination keys and at least one second combination key of the plurality of second combination keys to the verification unit;
comparing, by the verification unit, the at least one first combination key with the at least one second combination key; and
concluding the equality of at least one first secret code held by the first storage unit and at least one second secret code held by the second storage unit when the at least one first combination key equals the at least one second combination key, and
concluding the inequality of at least one first secret code held by the first storage unit and at least one second secret code held by the second storage unit when the at least one first combination key does not equal the at least one second combination key.

12. Method according to claim 11, comprising concluding the inequality of at least one first secret code held by the first storage unit and the plurality of second secret codes held by the second storage unit when the at least one first combination key does not equal any second combination key of the plurality of second combination keys.

13. Method according to any one of claims 10-12, comprising comparing, by the verification unit, each first combination key of the plurality of first combination keys with each second combination key of the plurality of combination keys; and
concluding the equality of at least one first secret code held by the first storage unit and at least one second secret code held by the second storage unit when at least one first combination key equals at least one second combination key, and
concluding the inequality of at least one first secret code held by the first storage unit and the plurality of second secret codes held by the second storage unit when at least one first combination key does not equal any second combination key of the plurality of second combination keys.

14. Method according to any one of the preceding claims, wherein the method comprises dividing the first secret code into a plurality of first secret subcodes and dividing the second secret code into a plurality of second secret subcodes,
generating by the first storage unit a plurality of first subkeys, each corresponding to a respective first secret subcode, and generating by the second storage unit a plurality of second subkeys, each corresponding to a respective second secret subcode;
transmitting the at least one first subkey from the first storage unit to the second storage unit;
transmitting the at least one second subkey from the second storage unit to the first storage unit;
generating by the first storage unit a plurality of first combination subkeys, each corresponding to a respective first secret subcode and second subkey, and generating by the second storage unit a plurality of second combination subkeys, each corresponding to a respective second secret subcode and first subkey;
transmitting at least one first combination subkey and at least one second combination subkey to the verification unit;
comparing, by the verification unit, the at least one first combination subkey with the respective at least one second combination subkey; and
concluding the equality of the first secret code and the second secret code unit when all first combination subkeys equal the respective second combination subkeys, and
concluding the inequality of the first secret code and the second secret code when at least one first combination subkey does not equal the respective second combination subkey.

15. Method according to any one of the preceding claims, wherein a plurality of first secret codes forms a first secret supercode and a plurality of second secret codes forms a second secret supercode, comprising
verifying the equality or inequality of at least one first secret code of the plurality of first secret codes to at least one respective second secret code of the plurality of second secret codes, until
the equality of all first secret codes with all respective second secret codes has been concluded, in which case the equality of the first secret supercode to the second secret supercode is concluded,
or until the inequality of at least one first secret code with the respective second secret code has been concluded, in which case the inequality of the first secret supercode to the second secret supercode is concluded.

16. Method according to any one of the preceding claims, wherein the verification unit, the first storage unit and the second storage unit are connectable via a network, such as the internet.

17. Method for determining whether an identical secret code is comprised in both a first privacy protected dataset and a second, optionally privacy protected, dataset, comprising:
verifying the equality of any first secret code comprised in the first dataset to any second secret code comprised in the second dataset, without revealing any first secret code and without revealing any second secret code.

18. System for verifying by a verification unit the equality or inequality of a first secret code without revealing the first secret code and the second secret code, comprising
a first storage unit holding the first secret code, a second storage unit holding the second secret code and a verification unit,
wherein the first storage unit is arranged for generating a first key from which the first secret code cannot be deduced, and for transmitting the first key to the second storage unit,
wherein the second storage unit is arranged for generating a second key from which the second secret code cannot be deduced, and for transmitting the second key to the first storage unit, for receiving the first key, for generating on the basis of the second secret code and the first key a second combination key, and for transmitting the second combination key to the verification unit,
wherein the first storage unit is further arranged for receiving the second key, for generating on the basis of the first secret code and the second key a first combination key, and for transmitting the first combination key to the verification unit,
wherein the verification unit is arranged for receiving the first combination key from the first storage unit, for receiving the second combination key from the second storage unit, for comparing the first combination key with the second combination key, and for concluding the equality of the first secret code held by the first storage unit and the second secret code held by the second storage unit when the first combination key equals the second combination key, and concluding the inequality of the first secret code held by the first storage unit and the second secret code held by the second storage unit when the first combination key does not equal the second combination key.

19. System according to claim 18 , wherein the first storage unit is further arranged for generating a third key on the basis of the first secret code and the first key, such that the first key and the third key in combination uniquely represent the first secret code,
wherein the second storage unit is arranged for generating a fourth key on the basis of the second secret code and the second key, such that the second key and the fourth key in combination uniquely represent the second secret code, and for generating the second combination key on the basis of the fourth key and the first key,
wherein the first storage unit is further arranged for generating the first combination key on the basis of the third key and the second key.

20. System according to claim 18 or 19, arranged for performing the method of any one of the claims 1-17.

21. Verification unit of the system according to any one of claims 18-20.

22. Storage unit of the system according to any one of claims 18-20.

23. Computer program product including program code portions for performing, when run on a programmable apparatus, a method according to any one of claims 1-17.
